# EUROPÄISCHE PATENTANMELDUNG

(11) **EP 1 240 944 A2**
(43) Veröffentlichungstag der Anmeldung: **18.09.2002**
(21) Anmeldenummer: 02003617.4
(22) Anmeldetag: 16.02.2002
(51) Int. Cl.: B01L 3/00, G01N 1/00

(54) **System zur Untersuchung von biologischen Flüssigkeiten**

(30) Priorität: 15.03.2001 DE 10112507
(71) Anmelder: Roche Diagnostics GmbH, 68298 Mannheim (DE); F. HOFFMANN-LA ROCHE AG, 4070 Basel (CH)
(72) Erfinder: Roesicke, Bernd, 68305 Mannheim (DE); Frey, Günter, 67158 Ellerstadt (DE)
(74) Vertreter: Pfeifer, Hans-Peter, Dr.

(57) **Zusammenfassung**

Disposable zur einmaligen Verwendung für die Untersuchung von biologischen Flüssigkeiten, insbesondere Zellsuspensionen wie Blut, Urin oder Sperma mit einer Verdünnungsflüssigkeitskammer (5), einer Probendosiereinrichtung (6) und eine Meßkammer (7). Die Probendosiereinrichtung (6) weist ein Dosierelement (8) auf, in das eine Dosierkapillare (13) integriert ist. Das Dosierelement (8) sitzt derartig beweglich in einer Dosierelementkammer (12), daß in einer ersten Position des Dosierelementes (8) eine Öffnung (14) der Dosierkapillare (13) mit einer Probenaufgabezone (17) des Disposables (2) in Verbindung steht und in einer zweiten Position die Verdünnungsflüssigkeitskammer (5) und die Meßkammer (7) über die Dosierkapillare (13) miteinander verbunden sind. Die Meßkammer (7) weist ein für Gas durchlässiges aber für die Probenflüssigkeit undurchlässiges Entlüftungsventil (35) auf, so daß sie von in sie eindringender Flüssigkeit (36) blasenfrei vollständig gefüllt wird.

## Beschreibung

Die Erfindung betrifft die Untersuchung von biologischen Flüssigkeiten, insbesondere Zellsuspensionen. Das wichtigste Anwendungsgebiet ist die Gewinnung hämatologischer Daten an Blut, jedoch kann die Erfindung vorteilhaft auch für die Untersuchung anderer Zellsuspensionen (insbesondere Urin oder Sperma) verwendet werden. Dabei geht es vor allem um die Untersuchung der Probe hinsichtlich der Zahl (pro Volumeneinheit) und Morphologie der in der Zellsuspension enthaltenen Zellen, jedoch kann die Erfindung grundsätzlich auch für andere analytische Untersuchungen an biologischen Flüssigkeiten verwendet werden. Nachfolgend wird ohne Beschränkung der Allgemeinheit auf das Anwendungsgebiet der Hämatologie Bezug genommen.

Traditionell werden hämatologische Daten an Blutproben visuell mittels Zellkammern gewonnen. Dieses Verfahren setzt eine sehr gute Schulung des Laborpersonals voraus. Auch soweit diese Voraussetzung erfüllt ist, ist es umständlich, langsam und fehlerträchtig.

Zur automatisierten Untersuchung der Blutproben werden Durchfluß-Zählgeräte eingesetzt, bei denen die Probenflüssigkeit - in der Regel nach vorausgehender Verdünnung - durch einen engen Kanal strömt und die Passage der Zellen mittels eines an dem Kanal angebrachten Impedanz-Detektors detektiert wird. Aus der Anzahl der Impulse, die von dem Detektor erzeugt werden, wenn Zellen die Meßstelle passieren, und dem Volumenstrom der Flüssigkeit lassen sich Zellzahlen ermitteln. Die Form der Impulse läßt sich zu Informationen über die Morphologie der Zellen verarbeiten. Neuerdings werden auch Durchfluß-Zählgeräte angeboten, bei denen statt eines Impedanz-Detektors ein optischer Detektor verwendet wird, bei dem die Detektion auf der Ablenkung eines Laserstrahls durch die Zellen basiert.

Da die Investitionskosten für derartige Geräte hoch sind und an die Qualifikation des Bedienungspersonals hohe Anforderungen gestellt werden, eignet sich dieses Verfahren nicht gut für lokale Anwendungen, insbesondere in der Arztpraxis oder dezentral auf Stationen von Krankenhäusern.

Auf dieser Basis liegt der Erfindung das Problem zugrunde, analytische Untersuchungen, insbesondere von biologischen Zellsuspensionen, derart zu vereinfachen, daß einerseits die erforderlichen Investitionskosten wesentlich reduziert werden und andererseits bei einfacher Handhabung eine gute Genauigkeit des analytischen Ergebnisses erreicht wird.

Die Aufgabe wird gelöst durch ein Disposable zur einmaligen Verwendung für die Untersuchung von biologischen Flüssigkeiten, insbesondere Zellsuspensionen wie Blut, Urin oder Sperma, enthaltend eine Verdünnungsflüssigkeitskammer, eine Probendosiereinrichtung und eine Meßkammer, wobei die Probendosiereinrichtung ein Dosierelement aufweist, in das eine zwischen zwei Öffnungen verlaufende Dosierkapillare integriert ist, und das Dosierelement derartig beweglich in einer in dem Disposable ausgebildeten Dosierelementkammer sitzt, daß in einer ersten Position des Dosierelementes eine Öffnung der Dosierkapillare mit einer Probenaufgabezone des Disposables in Verbindung steht und in einer zweiten Position eine der Öffnungen der Dosierkapillare mit der Verdünnungsflüssigkeitskammer und die andere Öffnung der Dosierkapillare mit der Meßkammer in Verbindung steht, so daß in der zweiten Position die Verdünnungsflüssigkeitskammer und die Meßkammer über die Dosierkapillare miteinander verbunden sind, und die Meßkammer ein definiertes Volumen hat und ein für Gas durchlässiges aber für die Probenflüssigkeit undurchlässiges Entlüftungsventil aufweist, so daß sie von in sie eindringender Flüssigkeit blasenfrei vollständig gefüllt wird.

Gegenstand der Erfindung ist auch ein System, umfassend Disposables der vorstehend erläuterten Art und ein Auswertegerät mit einer Halterung zur Positionierung eines Disposables in einer Meßposition, einem Betätigungselement, das auf die Verdünnungsflüssigkeitskammer derartig einwirkt, daß die darin befindliche Verdünnungsflüssigkeit unter Druck gesetzt wird und infolgedessen durch die Dosierkapillare in die Meßkammer strömt, wenn sich das Dosierelement in der zweiten Position befindet, einer Einrichtung zur Detektion einer physikalischen Eigenschaft von in der Meßkammer des in der Meßposition positionierten Disposables enthaltener Flüssigkeit und einer Auswerteeinrichtung zur Ermittlung eines Untersuchungsergebnisses aus dem Ergebnis der Detektion der physikalischen Eigenschaft.

Weiter richtet sich die Erfindung auch auf ein Verfahren zur Untersuchung von biologischen Flüssigkeiten mittels eines Disposables der vorstehend erläuterten Art, bei welchem die Probenflüssigkeit derart mit der Probenaufgabezone des Disposables in Kontakt gebracht wird, daß sie bei in der ersten Position befindlichem Dosierelement durch Kapillarkräfte in die Dosierkapillare gesaugt wird, das Dosierelement in die zweite Position verstellt wird, auf in der Verdünnungsflüssigkeitskammer befindliche Verdünnungsflüssigkeit derartig Druck ausgeübt wird, daß sie durch die Dosierkapillare in die Meßkammer strömt, wobei die Probenflüssigkeit aus der Dosierkapillare in die Meßkammer ausgespült wird, die Meßkammer von der aus der Dosierkapillare ausgespülten Probenflüssigkeit und der Verdünnungsflüssigkeit blasenfrei vollständig gefüllt wird, wobei das bei dem Füllvorgang von der in die Meßkammer eindringenden Flüssigkeit verdrängte Gas durch das für das Gas durchlässige, aber für die Probenflüssigkeit undurchlässige Entlüftungsventil entweicht und an der danach in der Meßkammer enthaltenen Flüssigkeit ("Untersuchungsflüssigkeit") eine physikalische Eigenschaft gemessen und zur Ermittlung des Untersuchungsergebnisses ausgewertet wird.

Der Begriff "Disposable" ist in der Medizintechnik zur Bezeichnung von zur einmaligen Verwendung vorgesehenen Elementen und Vorrichtungen gebräuchlich. Im Falle der vorliegenden Erfindung besteht das Disposable aus wenigen Teilen und läßt sich kostengünstig aus Kunststoff, insbesondere mittels eines Spritzgießverfahrens, herstellen.

Trotz dieser kostengünstigen Herstellung ist das Verdünnungsverhältnis der Probenflüssigkeit in der Verdünnungsflüssigkeit sehr genau vorherbestimmbar und reproduzierbar. Es ist nur von den Volumina der Dosierkapillare und der Meßkammer abhängig. Die Teile, die diese Volumina umschließen, können mit üblicher Kunststoff-Spritzgießtechnik mit exakt definiertem Hohlraumvolumen hergestellt werden.

Von besonderem Vorteil ist die Dosierung mittels einer im Strömungsweg hinter der Dosierkapillare angeordneten Meßkammer bei Anwendungsfällen, bei denen das Disposable die Verdünnungsflüssigkeit in der Verdünnungsflüssigkeitskammer herstellerseitig vorgepackt enthält. Selbst bei Verwendung eines weitgehend gasdichten Kunststoffmaterials ist innerhalb der in der Labortechnik erforderlichen langen Lagerzeiten (oft mehr als zwei Jahre) mit einem Verlust an vorgepackter Verdünnungsflüssigkeit aus der Verdünnungsflüssigkeitskammer zu rechnen. Im Rahmen der Erfindung kann dieser Verlust leicht dadurch ausgeglichen werden, daß das Volumen der Verdünnungsflüssigkeit in der Verdünnungsflüssigkeitskammer entsprechend größer als das Volumen der Meßkammer ist. Die Präzision der Analyse wird durch Verluste an Verdünnungsflüssigkeit während des Lagerns nicht beeinträchtigt.

Die praktische Erprobung hat gezeigt, daß man (mit einer Ausführungsform, bei der die Meßkammer mit einem Beobachtungsfenster zur Durchführung optischer Untersuchung versehen ist) auf einfache Weise die im Rahmen der Hämatologie erforderlichen Informationen über die Zahl und Morphologie der im Blut enthaltenen Zellen gewinnen kann. Die dabei erreichte hohe Genauigkeit zeigt, daß trotz der einfachen Konstruktion eine schnelle homogene Verteilung der gesamten in der Dosierkapillare enthaltenen Blutmenge in der Verdünnungsflüssigkeit erreicht wird.

Soweit die Erfindung für die Untersuchung von Zellsuspensionen verwendet wird, basiert das Untersuchungsergebnis auf einer mikroskopischen Beobachtung der nach dem Verdünnungsvorgang in der Meßkammer enthaltenen Flüssigkeit. Diese Beobachtung erfolgt vorzugsweise elektronisch mittels eines in das Beobachtungsmikroskop integrierten Bildwandlerchips und einer entsprechenden Auswerteeinrichtung. Entsprechende Techniken, die die Detektion und Auswertung der in der verdünnten Zellsuspension relativ schnell beweglichen Zellen ermöglichen, wurden für die automatische Überwachung des Wachstums von biochemischen Zellkulturen entwickelt. Sie werden unter anderem in folgenden Publikationen beschrieben:
1) DE 4032002 C2
2) H. Suhr et al. "In Situ Microscopy for On-Line Characterization of Cell-Populations in Bioreactors, Including Cell-Concentration Measurements by Depth from Focus", Biotechnology and Bioengineering, 1995, 106 bis 116
3) DE 19726518 A1
4) EP 0990936 A1
5) DE 19923074 A1

Das in diesen Publikationen beschriebene, als "In Situ-Mikroskopie (ISM)" bezeichnete Verfahren eignet sich auch für die vorliegende Erfindung. Insoweit wird deren Inhalt durch Bezugnahme zum Gegenstand der vorliegenden Anmeldung gemacht.

Die Erfindung wird nachfolgend anhand von in den Figuren dargestellten Ausführungsbeispielen näher erläutert. Die dargestellten und beschriebenen Besonderheiten können einzeln oder in Kombination miteinander eingesetzt werden, um bevorzugte Ausgestaltungen der Erfindung zu schaffen; es zeigen:
- Fig. 1: Eine stark schematisierte Prinzipdarstellung zur Erläuterung der Erfindung,
- Fig. 2: eine perspektivische Darstellung eines erfindungsgemäßen Disposables,
- Fig. 3: einen Längsschnitt durch ein Disposable gemäß Fig. 2,
- Fig. 4: einen Schnitt durch eine Meßkammer längs der Linie IV-IV in Figur 3.

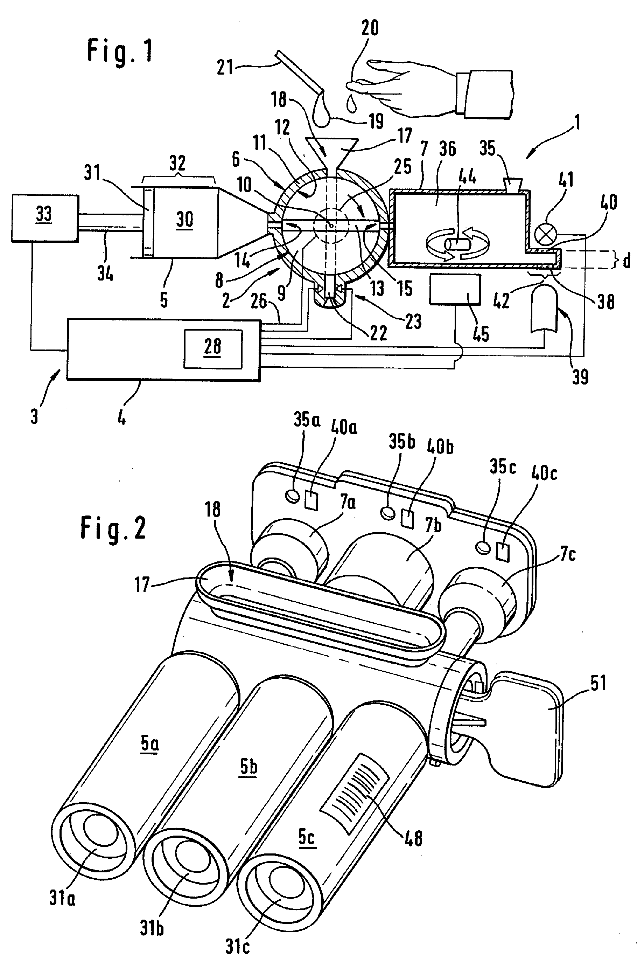

Das in Figur 1 symbolisch dargestellte System zur Untersuchung von Zellsuspensionen hinsichtlich der Zahl und Morphologie der darin enthaltenen Zellen eignet sich insbesondere zur Bestimmung hämatologischer Parameter (wie beispielsweise RBC, HCT, MCV, HGB, MPV und WBC) in Blutproben. Es wird nachfolgend als Hämatologiesystem 1 bezeichnet und besteht im wesentlichen aus einem Disposable 2, das zur Verdünnung und sonstigen Vorbereitungen der Probe dient und einem Auswertegerät 3 zur Durchführung einer optischen Messung und zur elektronischen Auswertung des Meßergebnisses. Das Auswertegerät enthält eine Meßund Auswerteelektronik 4 und verschiedene Vorrichtungen zur Halterung und Betätigung eines Disposables 2, die nachfolgend noch näher erläutert werden. Das Gehäuse des Auswertegerätes 3 ist der Übersichtlichkeit halber nicht dargestellt.

Bestandteile des Disposables 2 sind eine Verdünnungsflüssigkeitskammer 5, eine Probendosiereinrichtung 6 und eine Meßkammer 7. Die Probendosiereinrichtung 6 weist ein Probendosierelement 8 auf, das bei der dargestellten Ausführungsform als Rotorteil 9 ausgebildet ist und um eine Achse 10 drehbar in einer von einem Rotorgehäuse 11 gebildeten Dosierelementkammer 12 sitzt. In das Rotorteil 9 ist eine Dosierkapillare 13 mit zwei Öffnungen 14 und 15 integriert. Das Rotorteil 9 ist zwischen (mindestens) zwei Positionen drehbar, die sich hinsichtlich der Orientierung der Dosierkapillare 13 unterscheiden. In einer ersten Position steht eine Öffnung 14 der Dosierkapillare 13 derartig mit einer Probenaufgabezone 17 in Verbindung, daß eine dort vorhandene Blutprobe durch Kapillarkräfte getrieben in die Dosierkapillare 13 eindringt und diese vollständig füllt. Die Lage der Dosierkapillare 13 in dieser ersten Position des Rotorteils 9, die nachfolgend als Füllposition bezeichnet wird, ist in Figur 1 gestrichelt dargestellt.

Die Probenaufgabezone 17 kann unterschiedlich ausgebildet sein. Wesentlich ist nur, daß sie sich eignet, die Probe derartig zuzuführen, daß sie in die Dosierkapillare 13 eindringt, wenn sich das Rotorteil in der Füllposition befindet. Vorteilhaft ist eine Gestaltung in Form eines trichterförmigen Probenaufnahmeraums 18, der so gestaltet ist, daß er bequem (beispielsweise unmittelbar von einem mit einem Einstich versehenen Finger 20 oder mittels einer Pipettenspitze 21) mit der erforderlichen Menge einer Probenflüssigkeit 19 gefüllt werden kann.

Da die vollständige Füllung der Dosierkapillare 13 von großer Bedeutung für die Genauigkeit des analytischen Resultates ist, ist in dem Rotorgehäuse 11, an einer Stelle, die, wenn sich das Rotorteil 9 in der Füllposition befindet, mit der zweiten Öffnung 15 fluchtet, eine Kontrollkapillare 22 mit einer elektronischen Füllungskontrolleinrichtung 23, beispielsweise in Form einer Lichtschranke, vorgesehen. Die Füllungskontrolleinrichtung 23 zeigt an, wenn die Blutprobe in die Kontrollkapillare 22 eindringt und demzufolge die Dosierkapillare 13 vollständig gefüllt ist.

In der zweiten Position ("Durchflußposition") des Rotorteils 9 befindet sich die Dosierkapillare 13 in der mit durchgezogenen Linien dargestellten Position, in der sie einerseits (über die Öffnung 14) mit der Verdünnungsflüssigkeitskammer 5 und andererseits (über die Öffnung 15) mit der Meßkammer 7 in Verbindung steht, so daß die Verdünnungsflüssigkeitskammer 5 und die Meßkammer 7 über einen durchgängigen Flüssigkeitskanal miteinander verbunden sind.

Die Dosierkapillare 13 nimmt ein genau festliegendes Volumen an Probenflüssigkeit auf, das in die Meßkammer 7 gespült und für die gewünschte Untersuchung verwendet wird. Dieses Prinzip zur Dosierung eines definierten Volumens ist aus verschiedenen Laboranwendungen bekannt. Beispielsweise wird in der DE 3507032 C2 eine Vorrichtung zum volumenmäßigen Abmessen und Übergeben einer Probe von einem ersten Raum in einen zweiten Raum beschrieben, bei der die Dosierkapillare in einen beweglichen Übergabeschieber integriert ist, der in zwei unterschiedlichen Ausführungsformen entweder quer zur Längsrichtung der Dosierkapillare verschiebbar oder in einem entsprechenden Paßsitz zwischen unterschiedlichen Rotationspositionen drehbar ist. Wie bei dieser bekannten Vorrichtung kann auch bei der Erfindung das Dosierelement ein in einem passenden Gehäuse gleitend translatorisch verschiebbares Bauteil sein. Die in den Figuren dargestellte Gestaltung als Rotorteil ist jedoch bevorzugt.

Bei einem hämatologischen Durchfluß-Meßgerät mit Impedanzdetektion, das von der Firma SWELAB Instrument AB, Schweden, unter dem Namen "AutoCounter" vertrieben wird, wird ein sogenanntes Flüssigkeitsverteilerventil eingesetzt, in das ebenfalls eine Dosierkapillare integriert ist. Dieses Flüssigkeitsverteilerventil ist ein Hochpräzisionsbauteil aus Edelstahl und dient dazu, ein exakt reproduzierbares Probenvolumen von 20 µl in den Flüssigkeitsstrom einzudosieren.

Eine solche Vorrichtung ist auch in dem US-Patent 6,284,548 beschrieben. Die Verdünnungsflüssigkeit wird mittels einer im Strömungsweg vor der Dosierkapillare angeordneten hochpräzisen Kolben-Zylindereinheit mit einem vorbestimmten Volumen in die Dosierkapillare gedrückt. Eine solche Konstruktion ist für ein kostengünstig herstellbares Disposable ungeeignet.

Die Verdünnungsflüssigkeitskammer 5 enthält eine Verdünnungsflüssigkeit 30, die vorzugsweise herstellerseitig vorgepackt ist, also bereits von dem Hersteller des Disposables entsprechend dem gewünschten Anwendungszweck zubereitet und in die Verdünnungsflüssigkeitskammer 5 eingefüllt wurde, so daß das Disposable 2 ohne weitere Vorbereitungsschritte anwendungsbereit ist. Wie bereits dargelegt wurde, ist die Präzision der Verdünnung (und damit die Präzision der resultierenden analytischen Ergebnisse) nicht von einem etwaigen bei längerer Lagerzeit auftretenden Verlust an Verdünnungsflüssigkeit aus der Verdünnungsflüssigkeitskammer abhängig.

Die Verdünnungsflüssigkeitskammer 5 ist so ausgebildet, daß die darin befindliche Verdünnungsflüssigkeit 30 unter Druck gesetzt werden kann, so daß sie durch die Dosierkapillare 13 in die Meßkammer 7 strömt, wenn sich das Rotorteil 9 in der Durchflußposition befindet. Zu diesem Zweck weist die Verdünnungsflüssigkeitskammer 5 bei der dargestellten Ausführungsform einen beweglichen Kolben 31 auf, der in einem entsprechend zylindrisch ausgebildeten Gehäuseabschnitt 32 der Verdünnungsflüssigkeitskammer 5 axial verschiebbar ist. Als Betätigungselement zur Verschiebung des Kolbens 31 dient ein Linearantrieb 33, der Bestandteil des Auswertegerätes 3 ist, von dessen Meßund Auswerteelektronik 4 gesteuert wird und über eine Kolbenstange 34 auf den Kolben 31 wirkt. Im Rahmen der Erfindung können jedoch auch andere Druckerzeugungseinrichtungen für das Disposable verwendet werden. Insbesondere kann es zweckmäßig sein, die Wände der Verdünnungsflüssigkeitskammer 5 aus einem leicht deformierbaren Material (beispielsweise einer Kunststoffolie) herzustellen, so daß durch äußeren Druck auf die Wände ein entsprechender Druck in der Verdünnungsflüssigkeit 30 erzeugt werden kann. Die Genauigkeit der Dosierung wird durch diese kostengünstige Gestaltung nicht beeinflußt, weil sie von dem Volumen der Verdünnungsflüssigkeitskammer 5 unabhängig ist.

Wenn die Verdünnungsflüssigkeit 30 durch die Dosierkapillare 13 strömt, spült sie die darin befindliche Probenflüssigkeit vollständig in die Meßkammer 7. Die Meßkammer 7 hat ein für Gas durchlässiges, aber für die Probenflüssigkeit undurchlässiges Entlüftungsventil 35, das in der Benutzungsposition des Disposables an dem höchstgelegenen Teil der Meßkammer 7 angeordnet ist. Derartige Entlüftungsventile werden käuflich angeboten und in der Fachsprache auch als "hydrophobic vents" bezeichnet. Durch das Entlüftungsventil 35 wird sichergestellt, daß die Meßkammer blasenfrei vollständig gefüllt wird, so daß das am Ende des Füllvorganges in der Meßkammer 7 enthaltene Flüssigkeitsvolumen exakt'durch deren Rauminhalt bestimmt ist. Das Mischungsverhältnis aus Probenflüssigkeit 19 und Verdünnungsflüssigkeit 30 ist demzufolge durch das Volumen der Dosierkapillare 13 und durch das Volumen der Meßkammer 7 definiert. Die resultierende Mischung in der Meßkammer 7 wird nachfolgend als Untersuchungsflüssigkeit 36 bezeichnet.

Um eine optische Untersuchung der Untersuchungsflüssigkeit 36 zu ermöglichen, weist die Meßkammer 7 ein Beobachtungsfenster 38 auf, auf das eine lichtoptische Detektionseinrichtung 39 gerichtet ist. Zur Beleuchtung ist außerdem eine Lichtquelle 41 vorgesehen, die bei der dargestellten Ausführungsform durch ein gesondertes Beleuchtungsfenster 40 Licht in die Untersuchungsflüssigkeit 36 einstrahlt.

Gemäß einer bevorzugten Ausführungsform befindet sich das Beobachtungsfenster 38 in einem Meßbereich 42 der Meßkammer 7, in dem die Flüssigkeitsschicht - gemessen senkrecht zu der Fläche des Beobachtungsfensters 38 - eine im Vergleich zu der übrigen Meßkammer geringe Dicke hat. Bevorzugt beträgt die Dicke d der Flüssigkeitsschicht in dem Meßbereich höchstens 1 mm, besonders bevorzugt höchstens 0,5 mm. Der Volumenanteil der Flüssigkeit in dem Meßbereich beträgt bevorzugt höchstens ein Drittel, besonders bevorzugt höchstens ein Zehntel des Gesamtvolumens der Meßkammer 7.

Die optische Detektionseinrichtung 39 ist vorzugsweise ein elektronisches Mikroskop zur Beobachtung von in der Untersuchungsflüssigkeit enthaltenen Zellen. Besonders geeignet ist die In Situ-Mikroskopie (ISM), die aus den Literaturstellen 1) bis 5) bekannt ist.

Im Rahmen der Erfindung wurde festgestellt, daß sich das ISM-Verfahren in Verbindung mit dem dargestellten System sehr gut eignet, um hämatologische Parameter zu bestimmen. Im Gegensatz zu dem vorbekannten Zellkammerverfahren erfolgt die Bestimmung automatisch und es ist kein speziell geschultes Personal erforderlich. Im Gegensatz zu den vorbekannten Durchflußmeßgeräten ist der apparative Aufwand, der für das Auswertegerät 3 erforderlich ist, sehr gering, so daß ein derartiges System dezentral in Krankenstationen oder Arztpraxen wirtschaftlich eingesetzt werden kann.

Von großer Bedeutung für die Genauigkeit ist dabei, daß die Untersuchungsflüssigkeit 36 in der gesamten Meßkammer 7 einschließlich des Meßbereiches 42 so gut durchmischt wird, daß der Inhalt des Meßbereiches 42 für den Gesamtinhalt der Meßkammer 7 statistisch repräsentativ ist. Zu diesem Zweck ist eine Rühreinrichtung zum Rühren der in der Meßkammer 7 befindlichen Untersuchungsflüssigkeit 36 vorgesehen. Im dargestellten Fall ist in der Meßkammer 7 ein Magnetrührelement 44 enthalten, das von einem Magnetantrieb 45 in Rotation versetzt wird, der ein Bestandteil des Auswertegerätes 3 ist und von der Meß- und Auswerteelektronik 4 gesteuert wird. Alternativ können auch andere berührungslose Mischprinzipien verwendet werden, die geeignet sind, die Untersuchungsflüssigkeit 36 durch die geschlossene Wand der Meßkammer 7 in Mischbewegung zu versetzen, insbesondere ein Ultraschall-Mischer.

Wenn mit dem dargestellten System 1 eine Blutprobe 19 untersucht werden soll, wird sie in den trichterförmigen Probenaufnahmeraum 18 der Probenaufgabezone 17 eingebracht und dringt in die Dosierkapillare 13 ein, wobei sich das Rotorteil 9 in der Füllposition befindet. Wenn die Dosierkapillare 13 vollständig gefüllt ist, erzeugt die Füllungskontrolleinrichtung 23 ein Signal, das an das Auswertegerät 3 übermittelt wird. Anschließend wird das Rotorteil 9 in die zweite Position gedreht. Bevorzugt geschieht dies mittels eines elektrischen Rotationsantriebs 25, der in Figur 1 gestrichelt dargestellt ist und über eine Leitung 26 von dem Auswertegerät 3 gesteuert wird. Alternativ besteht auch die Möglichkeit, daß das Rotorteil 9 manuell gedreht wird, wobei der Benutzer durch eine entsprechende Anzeige auf dem Display 28 des Auswertegerätes 3 zum Drehen des Rotorteils 9 aufgefordert werden kann.

Wenn sich das Rotorteil 9 in der Füllposition befindet, wird der Linearantrieb 33 in Bewegung gesetzt und drückt Verdünnungsflüssigkeit 30 durch die Dosierkapillare 13 in die Meßkammer 7, wobei die verdrängte Luft durch das Entlüftungsventil 35 entweicht. Dieser Vorgang wird fortgesetzt, bis die Meßkammer 7 vollständig gefüllt ist. Zu diesem Zeitpunkt steigt der Druck in dem System und damit in der Verdünnungsflüssigkeitskammer 5 steil an. Dieser Druckanstieg kann in bekannter Weise detektiert und zum Abschalten des Linearantriebs 33 verwendet werden.

Schließlich wird die Rühreinrichtung 44,45 in Betrieb gesetzt, um die Untersuchungsflüssigkeit 36 in der Meßkammer 7 intensiv zu durchmischen und eine Messung, im bevorzugten Fall eine ISM-Untersuchung, daran vorzunehmen. Die Signale der lichtoptischen Detektionseinrichtung (39) werden an die Meß- und Auswerteelektronik übermittelt. Das Ergebnis der elektronischen Auswertung wird als Untersuchungsergebnis auf dem Display 28, per Datenübertragung oder auf andere Weise angezeigt.

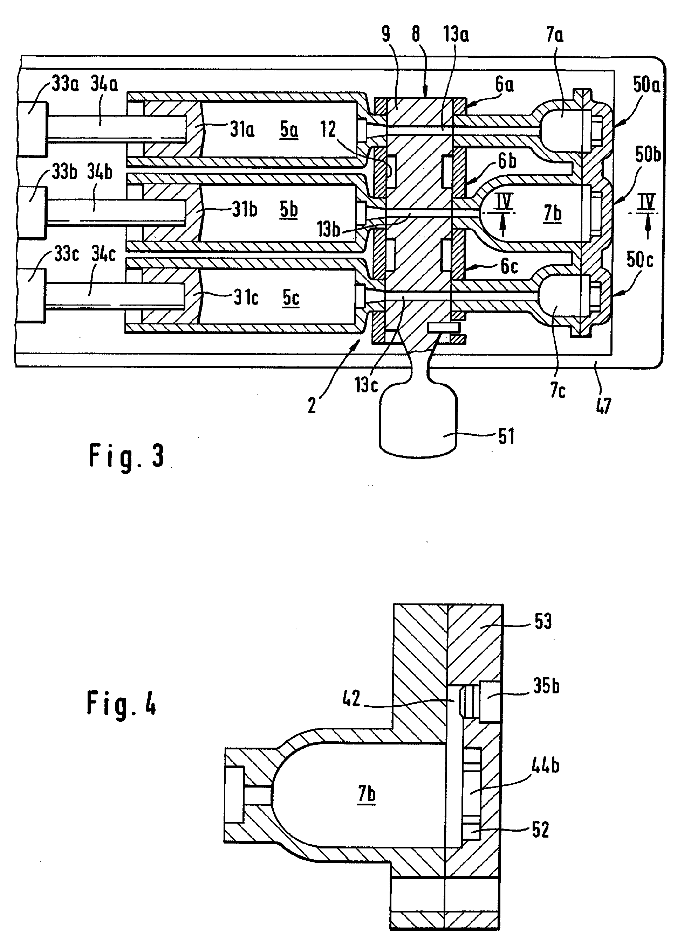

Die Figuren 2 bis 4 zeigen maßstabsgerechte Abbildungen eines Disposables 2, das zur praktischen Erprobung der Erfindung verwendet wurde. Elemente, deren Funktion mit denen der Figur 1 übereinstimmt, sind mit den gleichen Bezugsziffern bezeichnet. In Figur 3 ist außerdem schematisch eine Halterung 47 dargestellt, die Bestandteil eines Auswertegerätes 3 ist und in der ein Disposable 2 in einer Meßposition fixiert wird.

Eine Besonderheit des in den Figuren 2 bis 4 dargestellten Disposables besteht darin, daß es mehrere Meßkanäle 50a bis 50c aufweist, die je eine Verdünnungsflüssigkeitskammer 5a bis 5c, eine Probendosiereinrichtung 6a bis 6c und eine Meßkammer 7a bis 7c enthalten. Die Dosierkapillaren 13a bis 13c sind in einem gemeinsamen Dosierelement 8 integriert, das ebenso wie bei Figur 1 als Rotorteil 9 ausgebildet ist, welches in einer Rotorkammer 12 drehbar gelagert ist. Auch die übrigen für die Meßkanäle 50a bis 50c mehrfach vorhandenen Bauteile sind mit den Zusatzbuchstaben a bis c gekennzeichnet. Die Drehung des Rotorteils 9 zwischen der Füllposition (nicht dargestellt) und der Durchflußposition (dargestellt) erfolgt mittels eine Drehgriffes 51.

Dadurch, daß mehrere Meßkanäle 50a bis 50c in einem gemeinsamen Disposable 2 integriert sind, besteht die Möglichkeit, aus einer Probe unterschiedliche Reaktionsabläufe parallel durchzuführen, wobei die Verdünnungsflüssigkeitskammern 5a bis 5c in der Regel nicht nur eine inerte Flüssigkeit (insbesondere Wasser), sondern auch Reagenzbestandteile enthalten. Im Rahmen der Hämatologie kann es beispielsweise zweckmäßig sein, daß eine der Verdünnungsflüssigkeiten in den Verdünnungsflüssigkeitskammern ein Lysereagenz enthält, durch das die roten Blutkörperchen aufgelöst werden. Es kann auch zweckmäßig sein, daß sich die Meßkammern 7a bis 7c hinsichtlich ihrer Volumina unterscheiden, um unterschiedliche Verdünnungen zu realisieren. Vorzugsweise ist an dem Disposable ein maschinenlesbarer Code 48 angebracht, der zur Auswertung des Untersuchungsergebnisses wesentliche Informationen, insbesondere zur Identifikation des Inhaltes der Reagenzflüssigkeitskammern enthält.

Wie aus den Figuren 2 und 3 zu erkennen ist, hat das Rotorteil 9 eine zylindrische Außenfläche und sitzt in einer entsprechend zylindrisch ausgebildeten Rotorkammer 12. Unabhängig von der im Einzelfall gewählten Form des Dosierelementes 8 gilt, daß seine Oberfläche zumindest in der Umgebung der Öffnungen 14, 15 der Dosierkapillare 13 gleitend mit der sie umgebenden Oberfläche der das Dosierelement umgebenden Dosierelementkammer in Kontakt steht, daß die Dosierkapillare bei der Bewegung von der Füllposition zu der Durchflußposition verschlossen ist, so daß das in ihr enthaltene Flüssigkeitsvolumen während dieser Bewegung konstant bleibt.

In Figur 4 sind weitere Einzelheiten einer bevorzugten Konstruktion der Meßkammer zu erkennen. Das Magnetrührelement 44b sitzt vorzugsweise in einer entsprechenden Ausnehmung 52 des Meßkammer-Innenraums. Das Entlüftungsventil 35b ist in der dargestellten Meßposition an dem höchstgelegenen Teil der Meßkammer 7b angeordnet, der bei der dargestellten Ausführungsform zugleich den Meßbereich 42 bildet. Das ebenfalls in diesem Meßbereich 42 angeordnete Beobachtungsfenster ist in der Ebene des Deckelteils 53 in der Nachbarschaft des Entlüftungsventils 35b positioniert, aber in Figur 4 nicht zu erkennen, weil es nicht in der Schnittebene liegt.

## Patentansprüche

1. Disposable zur einmaligen Verwendung für die Untersuchung von biologischen Flüssigkeiten, insbesondere Zellsuspensionen wie Blut, Urin oder Sperma,
enthaltend
eine Verdünnungsflüssigkeitskammer (5), eine Probendosiereinrichtung (6) und eine Meßkammer (7),
wobei
die Probendosiereinrichtung (6) ein Dosierelement (8) aufweist, in das eine zwischen zwei Öffnungen (14,15) verlaufende Dosierkapillare (13) integriert ist,
das Dosierelement (8) derartig beweglich in einer in dem Disposable ausgebildeten Dosierelementkammer (12) sitzt, daß in einer ersten Position des Dosierelementes (8) eine Öffnung (14) der Dosierkapillare (13) mit einer Probenaufgabezone (17) des Disposables (2) in Verbindung steht und in einer zweiten Position eine der Öffnungen (14) der Dosierkapillare (13) mit der Verdünnungsflüssigkeitskammer (5) und die andere Öffnung (15) der Dosierkapillare (13) mit der Meßkammer (7) in Verbindung steht, so daß in der zweiten Position die Verdünnungsflüssigkeitskammer (5) und die Meßkammer (7) über die Dosierkapillare (13) miteinander verbunden sind, und
die Meßkammer (7) ein definiertes Volumen hat und ein für Gas durchlässiges aber für die Probenflüssigkeit undurchlässiges Entlüftungsventil (35) aufweist, so daß sie von in sie eindringender Flüssigkeit (36) blasenfrei vollständig gefüllt wird.

2. Disposable nach Anspruch 1, welches eine Mehrzahl von Meßkanälen (50a-50c) mit je einer Verdünnungsflüssigkeitskammer (5a-5c), einer Probendosiereinrichtung (6a-6c) und einer Meßkammer (7a-7c) enthält.

3. Disposable nach Anspruch 2, bei welchem mehrere Dosierkapillaren (13a-13c) der Probendosiereinrichtungen (6a-6c) in einem gemeinsamen Dosierelement (8) integriert sind.

4. Disposable nach einem der vorhergehenden Ansprüche, bei welchem das Dosierelement (8) als in der Dosierelementkammer (12) drehbar gelagertes Rotorteil (9) ausgebildet ist.

5. Disposable nach einem der vorhergehenden Ansprüche, bei welchem die Verdünnungsflüssigkeitskammer (5) mit einer herstellerseitig vorgepackten Verdünnungsflüssigkeit (30) gefüllt ist, wobei das Volumen der Verdünnungsflüssigkeit größer als das definierte Volumen der Meßkammer ist.

6. Disposable nach einem der vorhergehenden Ansprüche, bei welchem die Meßkammer (7) ein berührungslos angetriebenes, insbesondere magnetisches, Rührelement (44) enthält.

7. Disposable nach einem der vorhergehenden Ansprüche, bei welchem die Meßkammer (7) ein Beobachtungsfenster (38) zur Durchführung optischer Untersuchungen an einer in der Meßkammer (7) enthaltenen Flüssigkeit (36) aufweist.

8. Disposable nach Anspruch 7, bei welchem die Meßkammer (7) einen Meßbereich (42) mit einer Flüssigkeitsschicht aufweist, die von einem Teilvolumen der Meßkammer gebildet wird, wobei die Flüssigkeitsschicht im Vergleich zu der übrigen Meßkammer (7) ein kleines Volumen und eine geringe Dicke, gemessen senkrecht zu der Fläche des Beobachtungsfensters (38), hat.

9. Disposable nach Anspruch 8, bei welchem das Volumen der Flüssigkeitsschicht in dem Meßbereich (42) höchstens ein Drittel, bevorzugt höchstens ein Zehntel des Volumens der Meßkammer (7) beträgt.

10. Disposable nach Anspruch 8 oder 9, bei welchem die Dicke (d) der Flüssigkeitsschicht in dem Meßbereich (42) höchstens 1 mm, bevorzugt höchstens 0,5 mm beträgt.

11. Disposable nach einem der vorhergehenden Ansprüche, welches einen maschinenlesbaren Code (48) aufweist, der zur Auswertung des Untersuchungsergebnisses wesentliche Informationen enthält.

12. System für die Untersuchung biologischer Flüssigkeiten, insbesondere Zellsuspensionen wie Blut, Urin oder Sperma, umfassend Disposables (2) nach einem der vorhergehenden Ansprüche und ein Auswertegerät (3) mit
einer Halterung (47) zur Positionierung eines Disposables (2) in einer Meßposition,
einem Betätigungselement (33,34), das auf die Verdünnungsflüssigkeitskammer (5) derartig einwirkt, daß die darin befindliche Verdünnungsflüssigkeit (30) unter Druck gesetzt wird und infolgedessen durch die Dosierkapillare (13) in die Meßkammer (7) strömt,
wenn sich das Dosierelement (8) in der zweiten Position befindet,
einer Einrichtung (39) zur Detektion einer physikalischen Eigenschaft von in der Meßkammer (7) des in der Meßposition positionierten Disposables (2) enthaltener Flüssigkeit (36) und
einer Auswerteeinrichtung (4) zur Ermittlung eines Untersuchungsergebnisses aus dem Ergebnis der Detektion der physikalischen Eigenschaft.

13. System nach Anspruch 12, bei welchem das Auswertegerät ein Betätigungselement (25) zum Verstellen des Dosierelementes (8) zwischen der ersten Position und der zweiten Position aufweist.

14. System nach einem der Ansprüche 12 oder 13, bei welchem das Auswertegerät (3) eine Rühreinrichtung (45) zum Rühren von in der Meßkammer befindlicher Flüssigkeit aufweist.

15. System nach einem der Ansprüche 12 bis 14, bei welchem die Einrichtung (39) zur Detektion einer physikalischen Eigenschaft eine lichtoptische Detektionseinrichtung (39), insbesondere ein Mikroskop zur mikroskopischen Untersuchung von in der Meßkammer (7) befindlicher Flüssigkeit (36) einschließt.

16. Verfahren zur Untersuchung von biologischen Flüssigkeiten, insbesondere Zellsuspensionen wie Blut, Urin oder Sperma mittels eines Disposables (2) nach einem der Ansprüche 1 bis 11, bei welchem
die Probenflüssigkeit (19) derart mit der Probenaufgabezone (17) des Disposables (2) in Kontakt gebracht wird, daß sie bei in der ersten Position befindlichem Dosierelement (8) durch Kapillarkräfte in die Dosierkapillare (13) gesaugt wird,
das Dosierelement (8) in die zweite Position verstellt wird,
auf in der Verdünnungsflüssigkeitskammer (5) befindliche Verdünnungsflüssigkeit (30) derartig Druck ausgeübt wird, daß sie durch die Dosierkapillare (13) in die Meßkammer (7) strömt, wobei die Probenflüssigkeit (19) aus der Dosierkapillare (13) in die Meßkammer (7) ausgespült wird,
die Meßkammer (7) von der aus der Dosierkapillare (13) ausgespülten Probenflüssigkeit und der Verdünnungsflüssigkeit blasenfrei vollständig gefüllt wird, wobei das bei dem Füllvorgang von der in die Meßkammer (7) eindringenden Flüssigkeit verdrängte Gas durch das für das Gas durchlässige, aber für die Probenflüssigkeit undurchlässige Entlüftungsventil (35) entweicht und
an der danach in der Meßkammer (7) enthaltenen Flüssigkeit (36) eine physikalische Eigenschaft gemessen und zur Ermittlung des Untersuchungsergebnisses ausgewertet wird.

17. Verfahren nach Anspruch 16, bei welchem die Messung der physikalischen Eigenschaft eine mikroskopische Untersuchung der in der Meßkammer enthaltenen Flüssigkeit einschließt.

18. Verfahren nach Anspruch 17, bei welchem die mikroskopische Untersuchung mittels einer elektronischen Kamera erfolgt und das Bild der elektronischen Kamera elektronisch hinsichtlich der Zahl und Morphologie von in der Meßkammer (7) enthaltenen Zellen ausgewertet wird.
